# EUROPEAN PATENT APPLICATION

(11) **EP 4 847 616 A1**
(43) Date of publication of application: **02.12.2026**
(21) Application number: 25745445.4
(22) Date of filing: 14.01.2025
(51) Int. Cl.: C12N 15/113, A61K 31/7088, A61P 25/28

(54) **NOVEL APOE ANTISENSE OLIGONUCLEOTIDE AND USE THEREOF**

(30) Priority: 26.01.2024 KR 20240012016; 09.01.2025 KR 20250003337
(71) Applicant: Seoul National University R&DB Foundation, Seoul 08826 (KR)
(72) Inventor: MOOK-JUNG, Inhee, Seoul 03082 (KR); JUNG, Eun Sun, Seoul 03080 (KR); HAN, Jihui, Seoul 03080 (KR)
(74) Representative: Plougmann Vingtoft a/s
(86) International application number: PCT/KR2025/000770
(87) International publication number: WO 2025/159427

(57) **Abstract**

The present invention relates to: an apolipoprotein E (APOE) antisense oligonucleotide; and a pharmaceutical composition for treating Alzheimer's disease comprising same. The antisense oligonucleotide of the present invention can reduce the expression of the APOE4 variant gene that contributes to increased risk and exacerbation of Alzheimer's disease, and can be used as an RNA therapeutic agent for diseases caused by abnormal levels of APOE proteins or APOE variant genome expression.

## Description

### TECHNICAL FIELD

The present invention relates to an Apolipoprotein E (APOE) antisense oligonucleotide and a pharmaceutical composition including the same for preventing or treating Alzheimer's disease.

### BACKGROUND ART

Therapeutic agents that inhibit RNA expression are broadly divided into antisense oligonucleotides (ASOs) and RNA interference (RNAi). Among these, antisense oligonucleotides are single-stranded nucleic acid polymers having a specific sequence complementarity to target RNA and can regulate gene expression or influence mRNA splicing. When an ASO binds to the target site of pre-mRNA with a complementary sequence, an RNA-DNA heteroduplex is formed, and ribonuclease H (RNase H) is activated, cleaving the RNA strand through hydrolysis, thereby inhibiting the expression of the target protein through mRNA degradation. ASOs regulate the expression of genes and proteins through various mechanisms, which include not only mediating RNase H but also regulating splicing through the steric blocking of ribosomal subunits and targeting intron sites to interfere with the interaction of RNA-binding proteins, thereby affecting translation of proteins (Annu. Rev. Med. 2019. 70: 307-21). Currently, gapmer antisense oligonucleotides (ASOs), which mediate RNase H activity, are commonly used and have an advantage in that they exhibit activity in both the nucleus and the cytoplasm.

Since the FDA first approved the first antisense drug, VITRAVENE, in 1998 for the treatment of retinitis due to cytomegalovirus (CMV) infection in immunocompromised patients with HIV, and most recently in 2023, it granted accelerated approval to Biogen's QALSODY for adults with familial amyotrophic lateral sclerosis (ALS) who have a SOD1 mutation. This indicates that antisense oligonucleotides have the potential to treat diseases.

Meanwhile, apolipoprotein E (APOE) is a component of lipoproteins involved in cholesterol metabolism and lipid transport, and the APOE gene consists of three common alleles, E2, E3, and E4. Among them, APOE3 is the most common isoform, and the APOE4 variant is known to be the strongest genetic risk factor associated with the development of late-onset Alzheimer's disease that develops in people over 65 years of age (Neuron 63(3), 287-303, 2009). Despite the recognition of this risk, the development of drugs targeting lipid metabolism and APOE4 is still insufficient, even in the clinical and preclinical stages. As a strategy to block the negative effects of the APOE4 gene, the development of a therapeutic agent that delivers the APOE2 gene, which has a protective effect on the development of Alzheimer's disease, using recombinant viral vector technology is currently underway. However, no treatment to reduce the risk gene APOE4 is currently in clinical trials, and few studies have confirmed the therapeutic effects of antisense compounds that suppress or inhibit APOE expression in human cells for Alzheimer's disease. Therefore, new treatment options that can effectively reduce APOE4 expression are urgently needed.

### DISCLOSURE OF THE INVENTION

### TECHNICAL PROBLEM

Under the circumstances, the present inventors have studied an antisense oligonucleotide compound that targets the APOE transcript and induces a decrease in APOE protein expression, and as a result, have confirmed that the expression level of APOE can be reduced in APOE-expressing cell lines and in neurons, astrocytes, and brain organoids derived from human induced pluripotent stem cells homozygous for APOE4. In addition, the present inventors have confirmed that the amount of phosphorylated tau, known as a causative agent of Alzheimer's disease, is reduced by an antisense oligonucleotide targeting APOE in brain organoids generated from human induced pluripotent stem cells homozygous for APOE4, a causative gene that significantly increases the incidence of Alzheimer's disease; therefore, the present inventors have suggested that the compound may be beneficial for diseases related to abnormal APOE expression and activity and Alzheimer's disease patients homozygous for APOE4.

Accordingly, an object of the present invention is to provide an antisense compound, which includes a modified oligonucleotide consisting of 10 to 30 contiguously linked nucleotides that complementarily binds to a nucleic acid base sequence within a transcript of a gene encoding human Apolipoprotein E (APOE).

Another object of the present invention is to provide an antisense oligonucleotide having any one of SEQ ID NOS: 1 to 6, in which at least one nucleoside among the nucleosides included in the antisense oligonucleotide is a 2'-O-methoxyethyl (2'-MOE) modified nucleoside, and all internucleoside linkages are phosphorothioate linkages.

Still another object of the present invention is to provide a pharmaceutical composition for preventing or treating Alzheimer's disease including the antisense oligonucleotide.

### TECHNICAL SOLUTION

Specifically, the following descriptions are provided. Meanwhile, each description and embodiment disclosed in the present invention can also be applied to each other description and embodiment. That is, all combinations of the various elements disclosed in the present invention fall within the scope of the present invention. Furthermore, the scope of the present invention should not be considered limited by the specific descriptions described below.

An aspect of the present invention relates to an antisense oligonucleotide including at least one oligonucleotide selected from the group consisting of: an oligonucleotide of SEQ ID NO: 1 having 5'-GCATCCTGTGTGGAA-3'; an oligonucleotide of SEQ ID NO: 2 having 5'-GGACACTCACCTCAG-3'; an oligonucleotide of SEQ ID NO: 3 having 5'-GCCTGGCATCCTGTG-3'; an oligonucleotide of SEQ ID NO: 4 having 5'-CTCACCTCAGTTCCT-3'; an oligonucleotide of SEQ ID NO: 5 having 5'-CGCAGCCCACAGAACCTTCA-3'; and an oligonucleotide of SEQ ID NO: 6 having 5'-TGCAGGTCATCGGCATCGCG-3'.

The "oligonucleotide" of the present invention generally refers to a molecule including two or more covalently linked nucleosides, as defined by those skilled in the art. These covalently linked nucleosides may be referred to as nucleic acid molecules or oligomers. The oligonucleotide of the present invention may be chemically synthesized and may include one or more modified nucleosides or nucleotides.

The "antisense oligonucleotide" of the present invention may be defined as an oligonucleotide capable of regulating the expression of a target gene by hybridizing to a target nucleic acid, but is not limited thereto. The antisense oligonucleotide may be used interchangeably with an "antisense oligomer compound" or "ASO".

The antisense oligonucleotide of the present invention can complementarily bind to a nucleic acid base sequence within a transcript of a gene encoding human apolipoprotein E (APOE), and can include a modified oligonucleotide consisting of 10 to 30 consecutively linked nucleotides. In addition, the antisense oligonucleotide of the present invention can bind to an intron-exon or exon-intron boundary region of the exon 3 of human APOE pre-mRNA.

The antisense oligonucleotide of the present invention can reduce the expression of an APOE transcript and the expression of an APOE protein in human cells expressing APOE (*e.g.,* U373 and THP-1 cells) and neurons, astrocytes, or brain organoids derived from human induced pluripotent stem cells (iPSCs).

The antisense oligonucleotide of the present invention may include one or more modified nucleosides. The term "modified nucleoside" as used herein refers to a nucleoside that has been modified by the introduction of one or more modifications, compared to an equivalent DNA or RNA nucleoside. Specifically, the modified nucleoside of the present invention may include a modified sugar moiety and may be used interchangeably with a "nucleoside analog".

The modified nucleoside may be 2'-O-alkyl-RNA, 2'-O-methyl RNA (2'-OMe), 2'-alkoxy-RNA, 2'-O-methoxyethyl-RNA (2'-MOE), 2'-amino-DNA, 2'-fluoro-RNA, 2'-fluoro-DNA, arabino nucleic acid (ANA), 2'-fluoro-ANA, a bicyclic nucleoside analogue (locked nucleic acid (LNA)), but is not limited thereto. In addition, the modified form may be a form in which a phosphorothioate bond is introduced, a phosphorodiamidate morpholino oligonucleotide (PMO), a peptide nucleic acid (PNA), a locked nucleic acid (LNA), and a form modified at the 2' position of a ribose sugar, but is not limited thereto.

All nucleosides included in the antisense oligonucleotide of the present invention are 2'-O-methoxyethyl (2'-MOE) modified nucleosides, and all internucleoside linkages may be phosphorothioate linkages.

In addition, the antisense oligonucleotide of the present invention may be in the form of a gapmer. The gapmer has the design of NNNNNDDDDDDDDDDNNNNN, where N is a nucleoside variant and D is in the form of DNA, but the number of sequences may be modified. In particular, the nucleoside variant included in the antisense oligonucleotide may be selected from 2'-O-alkyl-RNA, 2'-O-methyl RNA (2'-OMe), 2'-alkoxy-RNA, 2'-O-methoxyethyl-RNA (2'-MOE), 2'-amino-DNA, 2'-fluoro-RNA, 2'-fluoro-DNA, arabino nucleic acid (ANA), 2'-fluoro-ANA, and a bicyclic nucleoside analog (locked nucleic acid (LNA)). The antisense oligonucleotide of the present invention may be designed to degrade a target mRNA by RNase H, and the antisense oligonucleotide may be in the form of a gapmer, but is not limited thereto.

One or more nucleosides among the nucleosides included in the antisense oligonucleotide of the present invention may be a 2'-O-methoxyethyl (2'-MOE) modified nucleoside, and all nucleoside linkages may be phosphorothioate linkages. In addition, one or more nucleosides at the 5'-terminus or one or more nucleosides at the 3'-terminus of the antisense oligonucleotide may be modified nucleosides, and specifically, the first to n^{th} nucleosides at the 5'-terminus or the first to m^{th} nucleosides at the 3'-terminus of the antisense oligonucleotide may be modified nucleosides. In particular, n and m are each independently any integer selected from 1 to 10.

The antisense oligonucleotide of the present invention may target various regions within the pre-mRNA and mRNA of human apolipoprotein E (APOE). Specifically, the antisense oligonucleotide of the present invention may have a sequence structure complementary to a sequence included in the human APOE gene, NCBI Reference Sequence: NC_000019.10 (SEQ ID NO: 7). The antisense oligonucleotide may target an intron-exon region (or an exon-intron region) in which splicing occurs to reduce the expression of the target gene and protein.

The apolipoprotein E (APOE) of the present invention is a fat-binding protein involved in fat metabolism in the body, and is known to be expressed in various organs, but especially in the liver and brain. APOE4, a variant type of the APOE gene, is known to be the strongest genetic risk factor related to the development of late-onset Alzheimer's disease. In this regard, the antisense oligonucleotide of the present invention was confirmed to inhibit the expression of the APOE4 variant gene, thus suggesting that it may be used in the treatment of diseases caused by the expression of the APOE variant gene. In addition, a recent study reported that cognitive and brain functions were affected when APOE4 was controlled in the liver, a peripheral tissue, rather than within the brain (Nat Neurosci. 2022 Aug; 25 (8): 1020-1033), and thus, the antisense oligonucleotide of the present invention has the advantage in that it can be delivered not only into the brain but also through peripheral tissues.

Another aspect of the present invention relates to a pharmaceutical composition for preventing or treating a disease caused by expression of an APOE variant gene, including an antisense oligonucleotide.

Diseases caused by the expression of the APOE variant gene specifically include Alzheimer's disease, coronary artery disease such as atherosclerosis, and hypercholesterolemia. Among these, Alzheimer's disease is preferred, but the present invention is not limited thereto.

The term "prevention" in the present invention refers to any action that inhibits or delays a disease by administering or treating a composition including the antisense oligonucleotide of the present invention as an active ingredient. The term "treatment" in the present invention refers to any action that improves or beneficially alters the symptoms of a disease by administering or treating a composition including the antisense oligonucleotide of the present invention as an active ingredient.

In an embodiment of the present invention, when the antisense oligonucleotide of the present invention was treated on a human induced pluripotent stem cell-derived brain organoid, it was confirmed that not only the expression of APOE protein but also the expression of phosphorylated tau, which is one of the causative proteins of Alzheimer's disease, was reduced, thus suggesting that the antisense oligonucleotide of the present invention can exhibit therapeutic activity on Alzheimer's disease even without using a separate carrier.

The pharmaceutical composition of the present invention may further include, in addition to the active ingredient, a pharmaceutically acceptable carrier, excipient, or diluent commonly used in the preparation of pharmaceutical compositions. Examples of the carrier, excipient, or diluent include, but are not limited to, water, saline, ethanol, polyol, lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinyl pyrrolidone, methylhydroxybenzoate, propylhydroxybenzoate, talc, magnesium stearate, mineral oil, and suitable mixtures thereof. The pharmaceutical composition of the present invention may be in the form of a solution, gel, suspension, emulsion, cream, ointment, suppository, patch, pad, or spray, but is not limited thereto. When formulated as described above, the composition may be prepared using a diluent or excipient, such as a filler, bulking agent, binder, wetting agent, disintegrant, and surfactant, commonly used in the art.

### ADVANTAGEOUS EFFECTS

The present invention relates to an antisense oligonucleotide that regulates APOE expression, which inhibits the expression of the APOE4 variant gene, a risk gene that influences the onset and worsening of Alzheimer's disease, and thus can be used to treat other diseases, including Alzheimer's disease, caused by abnormal levels of APOE protein or expression of APOE variant genes. Furthermore, the antisense oligonucleotide of the present invention is expected to have therapeutic effects not only through intracerebral delivery but also through peripheral delivery.

### BRIEF DESCRIPTION OF THE DRAWINGS

FIG. 1 is a schematic diagram of the human APOE gene and the antisense oligonucleotide (ASO) of the present invention targeting the same.
FIG. 2 is data confirming a decrease in APOE protein expression through ASO in U373 cells.
FIG. 3 shows data confirming a decrease in APOE mRNA expression through ASO in THP-1 cells.
FIGS. 4a to 4c are data confirming a decrease in APOE protein expression through ASO in THP-1 cells.
FIG. 5 shows data confirming a decrease in APOE mRNA expression through ASO without the use of a carrier in THP-1 cells.
FIG. 6 shows data confirming a decrease in phosphorylated tau protein along with a decrease in the expression of APOE mRNA and protein through ASO in human induced pluripotent stem cell (iPSC)-derived neuronal cells.
FIG. 7 is data confirming a decrease in APOE protein expression and a decrease in the amount of APOE protein secreted out of cells through ASO in human induced pluripotent stem cell-derived astrocytes.
FIG. 8 shows data confirming a decrease in APOE protein expression and phosphorylated tau protein through ASO in human induced pluripotent stem cell-derived brain organoids.

### MODE FOR CARRYING OUT THE INVENTION

Hereinafter, the present invention will be described in detail based on examples. However, the following examples are intended only to illustrate the present invention and are not intended to limit the spirit or scope of the present invention.

### Experimental Example 1. Construction of ASO

The antisense oligonucleotides (SEQ ID NOS: 1 to 4) described in the present invention are designed to target various regions within human APOE pre-mRNA and mRNA. Specifically, they target intron-exon regions (or exon-intron regions) where splicing occurs to reduce target gene and protein expression, thereby altering splicing outcomes by masking splicing signals and interfering with spliceosome recognition.

The ASO of SEQ ID NO: 1 was designed to target the 3' splice site of Exon3 (intron 10 mer + exon 5 mer), the ASO of SEQ ID NO: 2 was designed to target the 5' splice site of Exon3 (intron 10 mer + exon 5 mer), the ASO of SEQ ID NO: 3 was designed to target the 3' splice site of Exon3 (intron 5 mer + exon 10 mer), and the ASO of SEQ ID NO: 4 was designed to target the 5' splice site of Exon3 (intron 5 mer + exon 10 mer). SEQ ID NOS: 5 and 6 are Gapmer type ASOs that degrade target mRNA by RNase H. Therefore, the ASO of SEQ ID NO: 5 was designed to have a target sequence in Exon2 (5-10-5 type), and the ASO of SEQ ID NO: 6 was designed to have a target sequence in Exon4 (5-10-5 type). The designed ASOs were synthesized by Integrated DNA technologies.

**[Table 1]**

| SEQ ID NO: | ASO | 5'-sequence-3' | Length (mer) |
|---|---|---|---|
| 1 | ASO #1 | GCATC CTGTG TGGAA | 15 |
| 2 | ASO #2 | GGACA CTCAC CTCAG | 15 |
| 3 | ASO #3 | GCCTG GCATC CTGTG | 15 |
| 4 | ASO #4 | CTCAC CTCAG TTCCT | 15 |
| 5 | Gapmer #1 | CGCAG CCCAC AGAAC CTTCA | 20 |
| 6 | Gapmer #2 | TGCAG GTCAT CGGCA TCGCG | 20 |

More specifically, the ASOs of SEQ ID NOS: 1 to 4 have all nucleosides modified with 2'-O-methoxyethyl-RNA (2'-MOE), and all nucleosides are linked by phosphorothioate bonds. In addition, the gapmer ASOs of SEQ ID NOS: 5 and 6 have five nucleosides at both the 5' and 3' ends modified with 2'-O-methoxyethyl-RNA (2'-MOE), and all nucleoside linkages are in a form where phosphorothioate bonds are introduced.

### Experimental Example 2: Cell Culture and ASO Treatment

Human monocytic THP-1 cells and human astrocytoma U373 cells were cultured in DMEM medium containing 10% FBS and 0.1 mg/mL penicillin and streptomycin (P/S) at 37°C in a humidified 5% CO₂ atmosphere. For the experiment, 200,000 cells per well of a 12-well plate were cultured in 1 mL of medium. After about 24 hours of culture, the cells were mixed with ASO in opti-mem medium together with lipofectamine 2000 to a final concentration and transfected for 24 hours.

To differentiate neuronal progenitor cells (NPCs) derived from human induced pluripotent stem cells (iPSCs) into neurons, the cells were cultured in a Matrigel-coated environment in Neurobasal-A medium containing B-27 supplement and penicillin and streptomycin (P/S), with the growth factors BDNF and NT3. After 14 days of culture to generate mature neurons, the cultured cells were treated with ASO for 3 days.

To differentiate neuronal progenitor cells derived from human induced pluripotent stem cells into astrocytes, Y-27632 was added to the culture medium consisting of DMEM/F12, GlutaMAX (1:100), MEM-NEAA (Non-Essential Amino Acids) (1:100), B27 minus Vitamin A (1:50), N2 Supplement (1:100), recombinant human EGF (10 ng/mL), and recombinant human basic FGF (10 ng/mL) and cultured. After 24 hours, the culture medium was replaced with astrocyte culture medium, and this day was set as Day 0. The astrocyte culture medium is a Sciencell Cat#1801 product, consisting of AM Basal Medium + P/S (1:100) + FBS (1:50). Thereafter, all culture media were removed every 2-3 days and replaced with fresh culture media. Every 5-6 days, the cells were subcultured at a cell density of 15,000 cells/cm² using Accutase solution. On day 30, cells that had fully matured into astrocytes were collected and used for experiments. ASO was mixed with lipofectamine 2000 and transfected into astrocytes in opti-mem medium for 24 hours.

### Experimental Example 3. Preparation of Human Brain Organoids

To prepare brain organoids, human induced pluripotent stem cells homozygous for APOE4 were cultured in 24-well plates (AggreWell 800) in embryo body (EB) formation medium containing ROCK inhibitor Y-27632 on Day 0. The following day, the EB formation medium was replaced with Y-27632-free EB formation medium, and from Day 2, the medium was replaced daily for 3 days with DMEM/F-12 (containing GlutaMAX) supplemented with 20% KnockOut Serum Replacement (20%), 1% MEM Non-Essential Amino Acids Solution, 0.1 mM 2-mercaptoethanol, antibiotic P/S (100 U/mL), and 10 µM dorsomorphin and 10 µM SB-431542, which are SMAD inhibitors, to induce neural induction. On Day 6, individual embryo-like bodies were transferred one by one to ultra-low attachment 96-well plates and cultured in Neurobasal-A neural medium containing B-27 supplement, P/S, GlutaMAX, and 0.5% (v/v) Matrigel Basement Membrane Matrix, supplemented with EGF (20 ng/mL) and bFGF (20 ng/mL), and replaced daily until Day 24 to prepare organoids. From Day 25 to Day 42, the culture was replaced every other day with neural medium containing BDNF and NT-3 (each 20 ng/mL), and thereafter, the culture was maintained by replacing the medium with basic neural medium without Matrigel and growth factors once every 4 days. For ASO treatment, organoids cultured until Day 86 were used.

### Experimental Example 4. RNA Extraction and Quantitative Real-Time Reverse Transcription PCR

After incubation, the medium was removed, washed with PBS, and RNA was extracted using an RNA extraction kit (RNeasy Mini Kit, Qiagen) according to the manufacturer's instructions. The extracted RNA was quantified, and an equal amount of RNA was synthesized into cDNA using Maxime^{™} RT PreMix (iNtRON Biotechnology). Subsequently, quantitative real-time reverse transcription-PCR was performed using the KAPA SYBR Fast qPCR kit. The results were calculated as a percentage by comparing them to the negative control group.

### Experimental Example 5. Western Blot Analysis

The medium was removed, washed with PBS, and cells were harvested with RIPA buffer. Then, the cells were disrupted by sonication, and centrifuged at 4°C and 13,000 rpm for 15 minutes to obtain a supernatant. 10 µg of proteins were calculated by BCA assay and loaded onto a 4-12% Bis-Tris polyacrylamide precast gel (NuPAGE gel) for separation. Then, the proteins were transferred from the gel to a PVDF membrane, and then placed in a 5% skim milk solution and reacted at room temperature for 1 hour to prevent non-specific protein binding. Primary antibodies (APOE, AT8, pT181, PSD-95, and actin) were added thereto and reacted at 4°C for more than 16 hours. After the primary antibody reaction, the cells were washed 5 times with 1x PBST and HRP-conjugated secondary antibodies were reacted at room temperature for 1 hour. The signal was amplified with an ECL solution and developed using an Amersham^{™} Imager600 instrument, and the obtained protein band images were analyzed and quantified using MultiGauge software.

To analyze the APOE protein secreted outside the cells by Western blot, the conditioned medium of astrocytes was mixed with methanol/chloroform to precipitate the proteins present in the medium and used as a sample.

### Example 1. Confirmation of decreased expression of APOE protein following ASO treatment in U373 cells

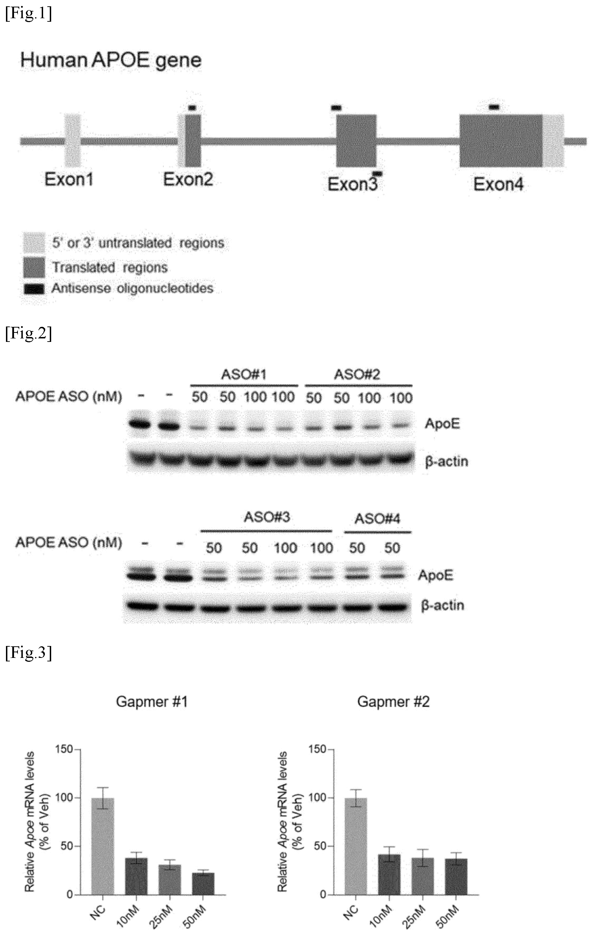

As a result of transfecting, U373 cells, which are human astrocytoma cells, with ASOs of SEQ ID NOS: 1 to 4 at a concentration of 50 nM or 100 nM using lipofectamine 2000 for 24 hours, it was confirmed that the expression of APOE protein was reduced compared to untreated cells (see FIG. 2).

### Example 2. Confirmation of decreased expression of APOE mRNA following ASO treatment in THP-1 cells

After transfecting THP-1 cells, which are human monocyte cells, with gapmer ASOs of SEQ ID NOS: 5 and 6 at concentrations of 10 nM, 25 nM, and 50 nM using lipofectamine 2000 for 24 hours, the expression of APOE mRNA was analyzed. As a result, it was confirmed that the expression of APOE mRNA was significantly reduced by the treatment with gapmer ASOs of SEQ ID NOS: 5 or 6 (see FIG. 3).

### Example 3. Confirmation of decreased expression of APOE protein following ASO treatment in THP-1 cells

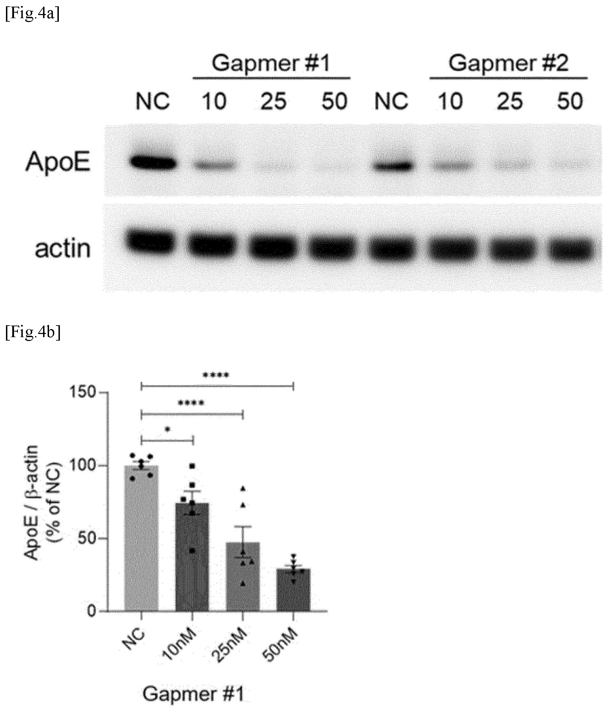

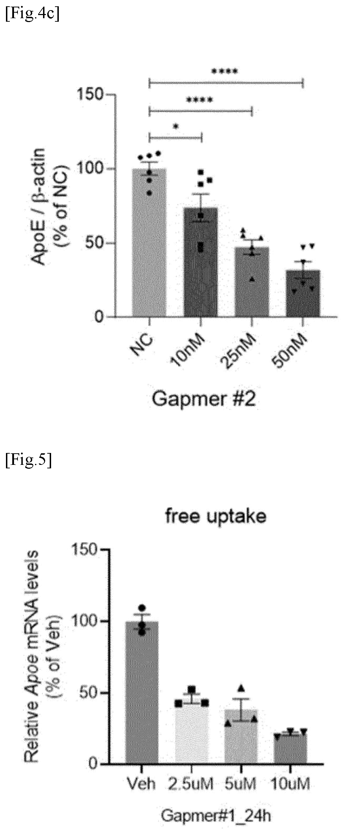

As a result of treating human monocyte THP-1 cells with gapmer ASOs of SEQ ID NOS: 5 and 6 at concentrations of 10 nM, 25 nM, and 50 nM, and then analyzing the expression of APOE protein, it was confirmed that the expression of APOE protein was decreased depending on the concentration of gapmer ASO treatment of SEQ ID NOS: 5 or 6 (see FIGS. 4a to 4c).

### Example 4. Confirmation of decreased expression of APOE mRNA by ASO treatment alone without use of carrier in THP-1 cells

After treating human monocyte THP-1 cells with ASO (Gapmer #1) of SEQ ID NO: 5 for 24 hours without the use of a carrier, the expression of APOE mRNA was analyzed. As a result, it was confirmed that the expression of APOE mRNA was decreased depending on the treatment concentration (see FIG. 5). This indicates that APOE ASO can enter cells and inhibit gene expression without the aid of a carrier.

### Example 5. Confirmation of decreased expression of APOE mRNA and protein through ASO treatment in human induced pluripotent stem cell (iPSC)-derived neuronal cells

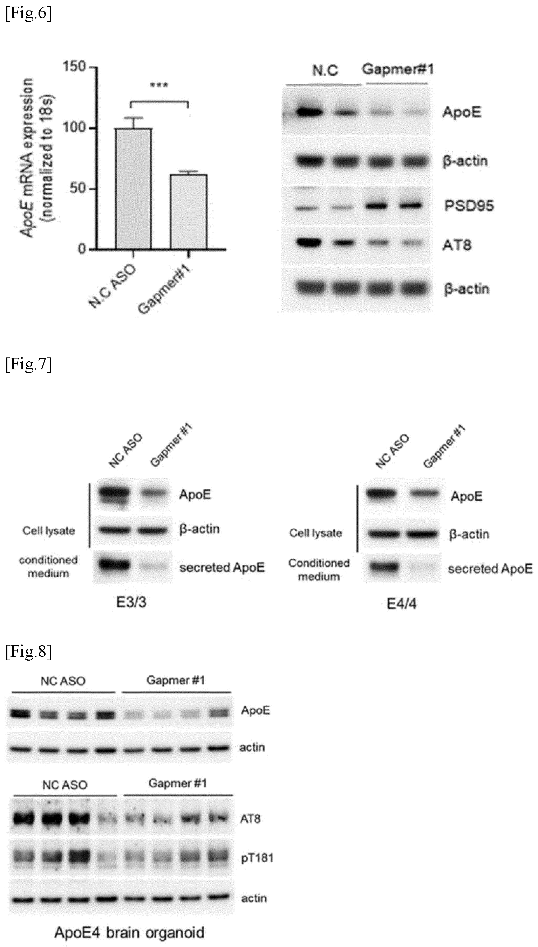

As a result of treating neuronal cells differentiated from human induced pluripotent stem cells homozygous for the APOE4 gene with ASO (Gapmer #1) of SEQ ID NO: 5 at a concentration of 5 µM for 3 days without using a carrier, it was confirmed that the amount of APOE mRNA and protein were decreased (see FIG. 6). In addition, as a result of confirming the amount of phosphorylated tau protein, which is a major pathogenic factor of Alzheimer's disease, with an AT8 antibody under conditions where APOE expression was reduced by ASO, it was observed that the amount was decreased, whereas the amount of PSD-95, which is the synaptic protein, was increased to the contrary.

### Example 6. Confirmation of decreased expression of APOE protein through ASO treatment in human induced pluripotent stem cell (iPSC)-derived astrocytes

Human induced pluripotent stem cell-differentiated APOE3-homozygous astrocytes and APOE4-homozygous astrocytes were transfected with 100 nM of ASO (Gapmer #1) of SEQ ID NO: 5 mixed with lipofectamine 2000 for 24 hours. The changes in APOE expression in astrocytes and the amount of APOE secreted from astrocytes were confirmed by Western blotting. The results of Western blot confirmed that the gapmer ASO of SEQ ID NO: 5 decreased the APOE protein expression in both astrocytes expressing APOE3 homozygous and APOE4 homozygous, respectively (see FIG. 7). In addition, as a result of measuring the amount of APOE protein released out of the cells by Western blotting, it was confirmed that the gapmer ASO of SEQ ID NO: 5 also decreased the amount of APOE protein released out of the cells (see FIG. 7).

### Example 7. Confirmation of decreased expression of APOE protein and phosphorylated tau protein through ASO treatment in human induced pluripotent stem cell (iPSC)-derived brain organoids

To confirm the potential as a treatment for Alzheimer's disease, human brain organoids were used. Brain organoids derived from human induced pluripotent stem cells homozygous for the APOE4 gene were treated twice at a rate of once every three days with ASO (Gapmer #1) of SEQ ID NO: 5 at a concentration of 10 µM without a carrier, and a decrease in APOE protein expression was confirmed (see FIG. 8). Furthermore, under conditions where APOE expression was reduced, the amount of phosphorylated tau, one of the causative proteins of Alzheimer's disease, was confirmed to be reduced in the group treated with ASO (Gapmer #1) of SEQ ID NO: 5 (see FIG. 8).

While specific aspects of the present invention have been described in detail above, it will be apparent to those skilled in the art that these specific descriptions merely represent preferred embodiments and are not intended to limit the scope of the present invention. Therefore, the substantial scope of the present invention is defined by the appended claims and equivalents thereof.

### [Sequence Listing]

SEQ ID NO: 1: APOE antisense oligonucleotide
   gcatcctgtg tggaa
SEQ ID NO: 2: APOE antisense oligonucleotide
   ggacactcac ctcag
SEQ ID NO: 3: APOE antisense oligonucleotide
   gcctggcatc ctgtg
SEQ ID NO: 4: APOE antisense oligonucleotide
   ctcacctcag ttcct
SEQ ID NO: 5: APOE antisense oligonucleotide
   cgcagcccac agaaccttca
SEQ ID NO: 6: APOE antisense oligonucleotide
   tgcaggtcat cggcatcgcg
SEQ ID NO: 7: Human APOE gene

ctactcagcc ccagcggagg tgaaggacgt ccttccccag gagccggtga gaagcgcagt
cgggggcacg gggatgagct caggggcctc tagaaagagc tgggaccctg ggaacccctg
gcctccaggt agtctcagga gagctactcg gggtcgggct tggggagagg aggagcgggg
gtgaggcaag cagcagggga ctggacctgg gaagggctgg gcagcagaga cgacccgacc
cgctagaagg tggggtgggg agagcagctg gactgggatg taagccatag caggactcca
cgagttgtca ctatcattta tcgagcacct actgggtgtc cccagtgtcc tcagatctcc
ataactgggg agccaggggc agcgacacgg tagctagccg tcgattggag aactttaaaa
tgaggactga attagctcat aaatggaaca cggcgcttaa ctgtgaggtt ggagcttaga
atgtgaaggg agaatgagga atgcgagact gggactgaga tggaaccggc ggtggggagg
gggtgggggg atggaatttg aaccccggga gaggaagatg gaattttcta tggaggccga
cctggggatg gggagataag agaagaccag gagggagtta aatagggaat gggttggggg
cggcttggta aatgtgctgg gattaggctg ttgcagataa tgcaacaagg cttggaaggc
taacctgggg tgaggccggg ttggggccgg gctgggggtg ggaggagtcc tcactggcgg
ttgattgaca gtttctcctt ccccagactg gccaatcaca ggcaggaaga tgaaggttct
gtgggctgcg ttgctggtca cattcctggc aggtatgggg gcggggcttg ctcggttccc
cccgctcctc cccctctcat cctcacctca acctcctggc cccattcagg cagaccctgg
gccccctctt ctgaggcttc tgtgctgctt cctggctctg aacagcgatt tgacgctctc
tgggcctcgg tttcccccat ccttgagata ggagttagaa gttgttttgt tgttgttgtt
tgttgttgtt gttttgtttt tttgagatga agtctcgctc tgtcgcccag gctggagtgc

agtggcggga tctcggctca ctgcaagctc cgcctcccag gtccacgcca ttctcctgcc
tcagcctccc aagtagctgg gactacaggc acatgccacc acacccgact aacttttttg
tattttcagt agagacgggg tttcaccatg ttggccaggc tggtctggaa ctcctgacct
caggtgatct gcccgtttcg atctcccaaa gtgctgggat tacaggcgtg agccaccgca
cctggctggg agttagaggt ttctaatgca ttgcaggcag atagtgaata ccagacacgg
ggcagctgtg atctttattc tccatcaccc ccacacagcc ctgcctgggg cacacaagga
cactcaatac atgcttttcc gctgggcgcg gtggctcacc cctgtaatcc cagcactttg
ggaggccaag gtgggaggat cacttgagcc caggagttca acaccagcct gggcaacata
gtgagaccct gtctctacta aaaatacaaa aattagccag gcatggtgcc acacacctgt
gctctcagct actcaggagg ctgaggcagg aggatcgctt gagcccagaa ggtcaaggtt
gcagtgaacc atgttcaggc cgctgcactc cagcctgggt gacagagcaa gaccctgttt
ataaatacat aatgctttcc aagtgattaa accgactccc ccctcaccct gcccaccatg
gctccaaaga agcatttgtg gagcaccttc tgtgtgcccc taggtactag atgcctggac
ggggtcagaa ggaccctgac ccaccttgaa cttgttccac acaggatgcc aggccaaggt
ggagcaagcg gtggagacag agccggagcc cgagctgcgc cagcagaccg agtggcagag
cggccagcgc tgggaactgg cactgggtcg cttttgggat tacctgcgct gggtgcagac
actgtctgag caggtgcagg aggagctgct cagctcccag gtcacccagg aactgaggtg
agtgtcccca tcctggccct tgaccctcct ggtgggcggc tatacctccc caggtccagg
tttcattctg cccctgtcgc taagtcttgg ggggcctggg tctctgctgg ttctagcttc
ctcttcccat ttctgactcc tggctttagc tctctggaat tctctctctc agctttgtct
ctctctcttc ccttctgact cagtctctca cactcgtcct ggctctgtct ctgtccttcc
ctagctcttt tatatagaga cagagagatg gggtctcact gtgttgccca ggctggtctt
gaacttctgg gctcaagcga tcctcccgcc tcggcctccc aaagtgctgg gattagaggc
atgagccacc ttgcccggcc tcctagctcc ttcttcgtct ctgcctctgc cctctgcatc
tgctctctgc atctgtctct gtctccttct ctcggcctct gccccgttcc ttctctccct
cttgggtctc tctggctcat ccccatctcg cccgccccat cccagccctt ctccccgcct
cccactgtgc gacaccctcc cgccctctcg gccgcagggc gctgatggac gagaccatga
aggagttgaa ggcctacaaa tcggaactgg aggaacaact gaccccggtg gcggaggaga
cgcgggcacg gctgtccaag gagctgcagg cggcgcaggc ccggctgggc gcggacatgg
aggacgtgtg cggccgcctg gtgcagtacc gcggcgaggt gcaggccatg ctcggccaga

gcaccgagga gctgcgggtg cgcctcgcct cccacctgcg caagctgcgt aagcggctcc
tccgcgatgc cgatgacctg cagaagcgcc tggcagtgta ccaggccggg gcccgcgagg
gcgccgagcg cggcctcagc gccatccgcg agcgcctggg gcccctggtg gaacagggcc
gcgtgcgggc cgccactgtg ggctccctgg ccggccagcc gctacaggag cgggcccagg
cctggggcga gcggctgcgc gcgcggatgg aggagatggg cagccggacc cgcgaccgcc
tggacgaggt gaaggagcag gtggcggagg tgcgcgccaa gctggaggag caggcccagc
agatacgcct gcaggccgag gccttccagg cccgcctcaa gagctggttc gagcccctgg
tggaagacat gcagcgccag tgggccgggc tggtggagaa ggtgcaggct gccgtgggca
ccagcgccgc ccctgtgccc agcgacaatc actgaacgcc gaagcctgca gccatgcgac
cccacgccac cccgtgcctc ctgcctccgc gcagcctgca gcgggagacc ctgtccccgc
cccagccgtc ctcctggggt ggaccctagt ttaataaaga ttcaccaagt ttcacgca

## Claims

1. An antisense oligonucleotide of any one of SEQ ID NOS: 1 to 4, wherein all nucleosides included in the antisense oligonucleotide are 2'-O-methoxyethyl (2'-MOE) modified nucleosides, and all internucleoside linkages are phosphorothioate linkages.

2. An antisense oligonucleotide of SEQ ID NO: 5 or 6, wherein one or more nucleosides among nucleosides included in the antisense oligonucleotide are 2'-O-methoxyethyl(2'-MOE) modified nucleosides, and all internucleoside linkages are phosphorothioate linkages.

3. The antisense oligonucleotide of claim 2, wherein one or more nucleosides at 5'-terminus or one or more nucleosides at 3'-terminus of the antisense oligonucleotide are modified nucleosides.

4. The antisense oligonucleotide of claim 3, wherein first to n^{th} nucleosides of the 5'-terminus or first to m^{th} nucleosides of the 3'-terminus of the antisense oligonucleotide are modified nucleosides (where, n and m are each independently any integer of 1 to 10).

5. The antisense oligonucleotide of claim 3, wherein first to fifth nucleosides of the 5'-terminus or first to fifth nucleosides of the 3'-terminus of the antisense oligonucleotide are modified nucleosides.

6. The antisense oligonucleotide of claim 1 or 2, wherein the antisense oligonucleotide is capable of reducing expression of Apolipoprotein E (APOE).

7. A pharmaceutical composition for the prevention or treatment of Alzheimer's disease comprising the antisense oligonucleotide of claim 1 or 2.
